# EUROPEAN PATENT SPECIFICATION

(11) **EP 2 130 519 B1**
(45) Date of publication and mention of the grant of the patent: **23.05.2012**
(21) Application number: 09161301.8
(22) Date of filing: 27.05.2009
(51) Int. Cl.: A61F 2/46

(54) **Device for preparing bone cement**
Vorrichtung zur Herstellung von Knochenzement
Dispositif pour préparer du ciment d'os

(30) Priority: 05.06.2008 GB 0810252
(43) Date of publication of application: 09.12.2009
(73) Proprietor: DePuy International Limited, Beeston, Leeds LS11 8DT (GB)
(72) Inventor: Chandler, Matt, Leeds, LS11 8DT (GB)
(74) Representative: Belcher, Simon James

(56) References cited:
- WO-A-2005/074840
- DE-U1- 29 607 832
- US-A- 4 971 068
- US-A- 5 806 528

## Description

This invention relates to a device for use in the provision of bone cement to a patient, for example in an orthopaedic surgery procedure. The device might be used in preparing bone cement or in delivering bone cement or in both.

Bone cement delivery devices in the form of syringes are disclosed in US-5328262, WO-93/22041, WO-94/26403 and WO-02/102287.

Bone cements that are used to provide fixation between bone tissue and an implanted prosthesis component (for example orthopaedic joint prosthesis components including spinal prostheses, and dental prostheses) are commonly provided by first and second materials which, when they react with one another, lead to the formation of a hard cement material. Examples of bone cement materials include those based on acrylate materials which can react by polymerising to form acrylate polymers. A bone cement composition can include acrylate polymer particles which react with monomer in the polymerisation reaction. A bone cement composition can also include other materials such as fillers, for example barium sulphate, zirconium dioxide, glass particles etc. A bone cement can be formed by mixing a liquid acrylate monomer with powders such as acrylate polymer particles and possibly barium sulphate, zirconium dioxide and/or glass particles. The resulting mixture has a paste or dough-like consistency. As is known, the components of the mixture react, involving polymerisation of the acrylate monomer and copolymerisation with the acrylate polymer particles. The viscosity of the cement composition increases during the reaction, resulting in a hard cement. The curing reaction of a bone cement material is generally exothermic.

It is known that surgical results can be optimised by ensuring that the cement is transferred from a mixing vessel to the prepared bone surface (for example, in a prepared bone cavity such as the intramedullary cavity in the femur or the humerus in the cases of a hip joint prosthesis or a shoulder joint prosthesis, or on a bone surface as in the case of a femur or a tibia in a knee joint prosthesis) where the prosthesis component is to be implanted when the cement is partially cured. The extent of the cure should exceed a minimum threshold so that the cement is not too fluid, facilitating handling of the cement and minimising the risk of the cement flowing undesirably after having been placed in contact with the prepared surface of the bone. As discussed below, the time taken to reach this stage in the cure reaction can be referred to as the End of Waiting Time. However, the extent of the cure should not exceed a maximum threshold, in order that subsequent introduction of the prosthesis component is not compromised, and in order that the cement should be able to penetrate the porous surface structure of the bone tissue. The time taken to reach this stage in the cure reaction can be referred to as the End of Working Time.

It is also known that surgical results can be optimised by ensuring that the prosthesis component is placed in contact with the bone cement (for example in a prepared bone cavity such as the intramedullary cavity in the femur or the humerus in the cases of a hip joint prosthesis or a shoulder joint prosthesis, or on a bone surface as in the case of a femur or a tibia in a knee joint prosthesis) when the cement is partially cured (with the extent of cure being greater than the extent of cure when the cement is placed on the prepared bone surface). The extent of the cure should exceed a minimum threshold so that the cement is not too fluid, which could mean that the cement would flow to an undesirable degree when the prosthesis component is deployed. However, the extent of the cure should not exceed a maximum threshold, in order that introduction of the prosthesis component and the formation of a bond to the component are not compromised.

The temperature of a bone cement material changes as the components of the material react, and this change in temperature can continue in the material after it has been placed in contact with a patient's bone. Monitoring the change in the temperature of the bone cement material can provide an indication of when the cement material has cured sufficiently for an implant to be secured against movement, for example to allow a surgeon to release the implant and to proceed with another stage of the surgical procedure. This is sometimes referred to as the Final Setting Time.

It is common for a surgeon to determine the extent of cure by feel, involving kneading the cement as it cures and relying on judgement to assess whether the extent of cure of the cement has reached such a level that it is appropriate to transfer the cement to the prepared bone surface, and to such a level that it is appropriate subsequently to introduce the prosthesis component to the cement. Features of the cement which characterise its extent of cure include viscosity (or firmness), tackiness, and smoothness ("grittiness"). Assessment of these features can be affected by environmental conditions. The speed of cure is affected by the temperature of the cement components when they are mixed and on ambient temperature. A factor such as the perceived tackiness can be affected by temperature and humidity.

Relying on subjective techniques such as feel to determine the extent of cure has the disadvantage that it is not always reliable, and it can be difficult to train new users of these techniques. Furthermore, the nature of the cure reaction is such that it can be affected by variations in conditions, especially temperature. Notably, variations in the temperature of the surgeon's fingers as he kneads a sample of a bone cement can lead to variations in the extent of cure of that sample, relative to the extent of cure of the remainder of the cement which is to be used in the procedure. Also, variations in temperature and humidity will affect the perception of the tackiness of the cement.

EP-A-380867 discloses a bone cement mixing system which uses a cartridge mixing device. The extent of cure of the cement can be monitored by placing a small quantity of the cement in a container whose wall is formed by a temperature sensitive tape.

The closest prio art is disclosed in DE-U-296 07832.

The present invention provides a device for use in the provision of bone cement to a patient, which comprises a syringe body and a tube through which cement can be supplied to a bone cavity, in which the tube comprises a polymer which is loaded with a thermochromic material.

In another aspect, the invention provides a device as discussed above in combination with a quantity of a bone cement material.

The thermochromic material can provide an indication of the extent of cure of a bone cement material. The indication will involve a change in the colour of the device, caused by exposure of the component to a change in temperature of the bone cement material as it cures. The temperature at which the change in the colour of the thermochromic material takes place can be selected by appropriate selection of the material. The thermochromic material can be selected to indicate a colour change when the temperature of the bone cement material is characteristic of, for example, the End of Waiting Time or the End of Working Time or the Final Setting Time of the cement material. A plurality of different thermochromic materials can be used to indicate temperatures which are characteristic of different stages in the cure of a cement material.

Thermochromic materials which can be used in the device of the invention can change between two colours, or between a coloured condition and colourless. A thermochromic material can be characterised by an activation temperature, which is the temperature at which the material has reached its final colour (or clear) state. The colour change has been found to take place when the sensed temperature increases towards the activation temperature over a small range of temperatures extending from about 4°C below to the activation temperature to the activation temperature.

Suitable materials for use as the thermochromic material are available from B&H Colour Change Limited of London GB-SW18 2RU.

The device of the invention can include a second indicator component formed from a polymer which is loaded with a second thermochromic material. The first and thermochromic materials can have different activation temperatures, for example to indicate different stages in the curing reaction of the bone cement material.

Preferably, the polymer which is loaded with the thermochromic material is used to form the tube by a moulding process (a process which involves the application of heat and pressure). Consequently, the thermochromic material cannot be separated from the material of the tube. For example, the tube might be formed by an extrusion process. The forming of the tube might include a drawing step, for example to impart a taper to the tube. Additional steps in the manufacture might be relied on to provide fixation fittings (for example a threaded fastener) so that the tube can be fastened to other components.

The thermochromic material can be provided in a separate piece which can cooperate with the wall of the tube, in particular by being placed in appropriate intimate contact with the wall of the tube so that a change in the temperature of cement material within the tube can be sensed by the thermochromic material. The indicator component with its thermochromic material can be fastened to the wall of the tube, for example by means of a bonding material or mechanically, for example by means of pins or screws. It could also be held in contact with the wall of the tube by surface tension effects, for example with the indicator component with its thermochromic material is in the form of a thin film which can be wrapped around at least part of the outer surface of the tube.

The polymeric material which contains the thermochromic material can be used to form all of the tube. Preferably, the amount of the thermochromic material in the polymer differs between different regions of the tube. For example, the thermochromic material can be provided in one or more stripes extending along at least part, preferably all, of the length of the tube. For example, at least two stripes, preferably at least three or at least four stripes, might be arranged around the circumference of the tube. The provision of the in thermochromic material indicator in one or more stripes which extend along the tube has the advantage that a change in the temperature of the cement along the length of the tube can be observed. The thermochromic material which is provided in a first stripe can be different from the thermochromic material which is provided in another of the stripes. Three or more different indicator materials might be used to form different ones of the stripes. The different indicators can provide indications of different extents of cure of the cement. For example, the indicator which provides one of the stripes might change colour to indicate that the cement has cured sufficiently for it to be ready to be injected into the bone cavity. The indicator which provides one of the stripes might change colour to indicate that the cement has cured so much that it should not be worked any further. The indicator which provides one of the stripes might change colour to indicate that the cement has set.

Preferably, the amount of thermochromic material in the material of the tube between first and second ones of the stripes is less than the amount of thermochromic material in the said first and second stripes. Preferably, one or more portions of the wall of the tube contain relatively small amounts of thermochromic indicator material, more preferably approximately none (apart perhaps from trace quantities). This can enhance the visibility of the bone cement within the tube when the material of the tube is at least partially transparent (for example translucent). This can be useful for the user of the device, for example to inspect for voids in the cement.

An advantage of incorporating the thermochromic material into the wall of a tube through which cement is supplied to a bone cavity is that the thickness of the cement can be comparable with the thickness of the cement within the bone cavity. Accordingly, the thermal behaviour of the cement in the tube during the cure process will be comparable with that of the cement in the bone cavity.

The syringe body can include a piston by which cement components in the syringe body can be mixed so that the cement will then cure, ready for use.

The syringe can include a piston by which mixed cement can be displaced from within the syringe body. The piston which is used to cause cement to be displaced from within the syringe body can be the same as part or all of the piston which is used to mix the cement. Alternatively the syringe can include separate pistons for mixing and delivering the cement. The syringe can be supplied with cement which has already been mixed, for delivery to a bone cavity.

The indicator component can be provided in a separate piece which can cooperate with the wall of the supply tube, in particular by being placed in appropriate intimate contact with the tube so that a change in the temperature of cement material within the tube can be sensed by the indicator component. The indicator component can be fastened to the wall of the tube, for example by means of a bonding material or mechanically, for example by means of pins or screws. The indicator component can be in the form of a collar which is positioned around the tube. It can be held in place as a result of having been stretched to fit it around the tube, and of the resulting forces as it attempts to recover towards its unstretched condition. It could also be held in contact with the wall of a tube by surface tension effects, for example with the indicator component in the form of a thin film which can be wrapped around at least part of the tube.

Polymers which can be used in the indicator component can include polyolefins, polyamides, polyesters. It will frequently be preferred that the polymer is flexible to facilitate handling. Examples of preferred polymers include ethylene polymers and propylene polymers.

The thermochromic material can be combined with the polymer of the indicator component as a powder. Particles of the thermochromic material can include a thermochromic dye material which is encapsulated in a polymeric carrier (for example comprising a low density polyethylene). For example, a thermochromic material can be provided in the form of microcapsules which contain crystal violet lactone, a weak acid, and a dissociable salt dissolved in a non-polar or slightly polar solvent liquid crystal solvent such as dodecanol or another suitable liquid crystal solvent. When the mixture is a solid, the dye exists in its lactone leuco form. However, when the liquid crystal solvent melts, the salt dissociates, the pH inside the microcapsule lowers (making protons readily available), the dye becomes protonated, and the lactone ring opens causing its absorption spectrum to shift, absorbing in the visible spectrum, such as a deeply violet colour for crystal violet lactone.

Suitable thermochromic dyes can be based on mixtures of leucodyes with suitable other chemicals, which display a colour change (usually between a colourless leuco form and the coloured form of the dye) dependent on the temperature. The dyes can be applied on the bone cement directly.

Examples of thermochromic materials which can be used in the device of the invention include spirolactones, fluorans, spiropyrans, and fulgides. Weak acids that can be used as proton donors include bisphenol A, parabens, 1,2,3-triazole derivatives, and 4-hydroxy-coumarin. These weak acids can function as a proton donor to cause a dye molecule to change between its leuco form and its protonated coloured form. Stronger Brönsted acids (better proton donors) can also be used but they tend to make the colour change irreversible. Other thermosensitive dyes that can be used include an oxazine-based leuco thermosensitive dye (such as that sold under the trade mark CSB-12 by Hodogaya Chemicals Co), a spiropyran-based leuco thermosensitive dye (such as that sold under the trade mark CSR-13 by Hodogaya Chemicals Co), a quinoline-based thermosensitive dye (such as that sold under the trade mark CSY-13 by Hodogaya Chemicals Co) and the like.

A plurality of thermosensitive dyes are known and are available commercially. The thermosensitive dyes are not particularly limited, but it is desired that dyes that are not toxic are used. A plurality of thermosensitive dyes are available that change colours at a variety of temperatures. Suitable thermochromic dyes are known which activate at temperatures in the range of 21 to 51 °C and which are available from SICPA Securink Corporation of Springfield, VA. These dyes include 744020TC (thermochromic blue), 744010TC (thermochromic turquoise), 744027TC (thermochromic yellow), 734010TC (thermochromic rose), 724010TC (thermochromic orange), 754027TC (thermochromic green). There are also thermochromic dyes which lose colour when heated, so that they change from a colour towards clear. These dyes include 178002TC (black/clear) which is active at 27 to 36°C. Compounds which are active at 22 to 31°C include 128001TC (orange/clear), 1384175TC (rose/clear), 150015TC (green/clear), 148003TC (blue/clear), 17800TC (black/clear), 14001TCBR (blue/red) and 128001TCY (orange/yellow). Compounds which are active from 24 to 33°C include 118000TC (yellow/clear), 128002TC (orange/clear), 138103TC (vermillion/clear), 15002TC (green/clear), 14001TC (blue/clear), 14000TCBR (blue/red) and 128001 TCY (orange/yellow). Compounds which are active at 24 to 33°C include 11800TC (yellow/clear), 128002TC (orange/clear), 138103TC (vermillion/clear), 15002TC (green/clear), 14001TC (blue/clear), 14000TCBR (blue/red) and 128002TC (orange/yellow). Compounds which are active at 32 to 41°C include 13001TC (rose/clear), 148002TC (blue/clear), 178001TC (black/clear) and 178002TCBR (blue/red). The compound should be non-toxic. It is an advantage of the device of the present invention that the compound does not form part of a component or a composition which is implanted in a patient. This can reduce the risk of an adverse reaction as a result of contact with a patient's tissue or body fluid.

It will often be preferred that a change in the colour of the thermochromic material be detected by visually inspecting the colour change. Alternatively, a spectrophotometer or some other optical sensor instrument can be used to detect the colour change. It can however be preferred to use an optical sensor, for example to provide greater precision, or to differentiate between small changes in colour. Moreover, using an instrument that can detect colour change allows one to find the optimum extent of cure when tints of various colours are detected (i.e., small changes on the pantone scale).

The concentration of the thermochromic material in the polymer of the indicator component will be selected to provide an adequately visible colour change response. The composition of the material of the indicator component should also take into account characteristics such as the desired physical properties of the component.

Details of the invention are described below by way of example with reference to the accompanying drawings, in which:
Figure 1 is graph which shows the variation of temperature during the course of the cure of a bone cement material.
Figure 2 is a side view of part of a first embodiment of syringe in which the delivery tube for the cement includes stripes of a thermochromic indicator material.
Figure 3 is an isometric view of a second embodiment of nozzle of a cement delivery syringe which incorporates a thermochromic material.
Figure 4 is an isometric view of a third embodiment of nozzle of a cement delivery device which incorporates a thermochromic material.

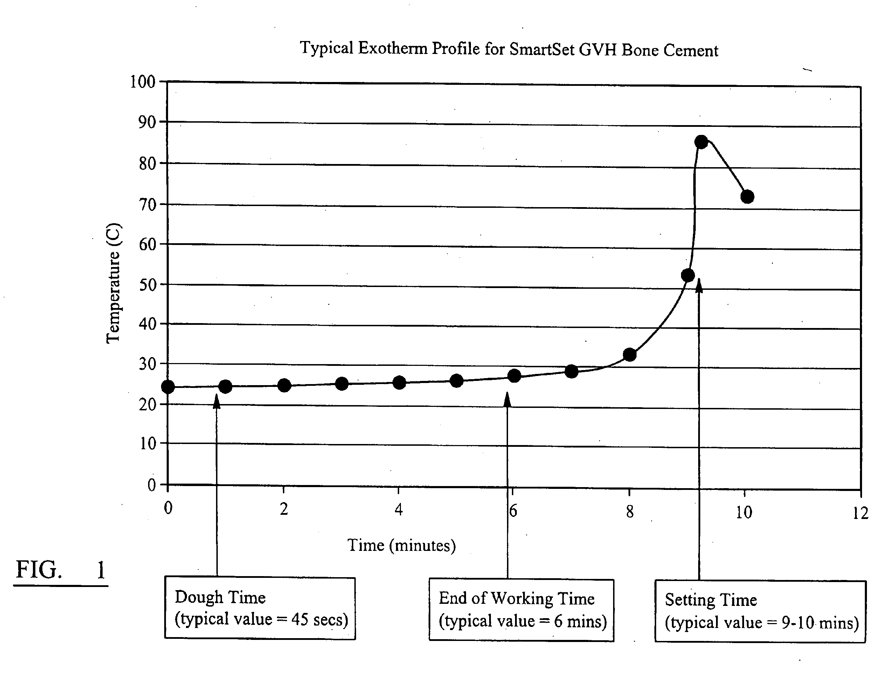

Referring to the drawings, Figure 1 is a graph which shows how the temperature of a bone cement varies during the period after powder and liquid components are mixed. The cement which was used to generate the data for Figure 1 is that sold by DePuy International Limited under the trade mark SmartSet GHV. It comprises copolymers based on methylmethacrylate and methylacrylate groups, with added methylmethacrylate monomer, ZrO₂ radiopaque agent and gentamicin sulphate antibiotic (optionally). The components of the cement were stored at 24°C before being mixed. They were mixed in a container which had been stored at 24°C. The variation in temperature was monitored from the time when the components of the cement were mixed. It can be seen that the temperature of the cement increased slowly over an initial period of about 7 minutes. The temperature of the cement then increased at a steadily increasing rate, reaching a maximum of about 86°C after about 9 minutes. The maximum temperature of the cement has been found to be attained at about the end of the period over which the cement sets so that, at the end of the period, an implant component of a joint prosthesis is fixed to a bone. It is at this stage that the surgeon can move on from the stage of the procedure in which that component is fixed to the bone to another stage. Information as to when an implant component is adequately fixed to a bone can be important for a surgeon.

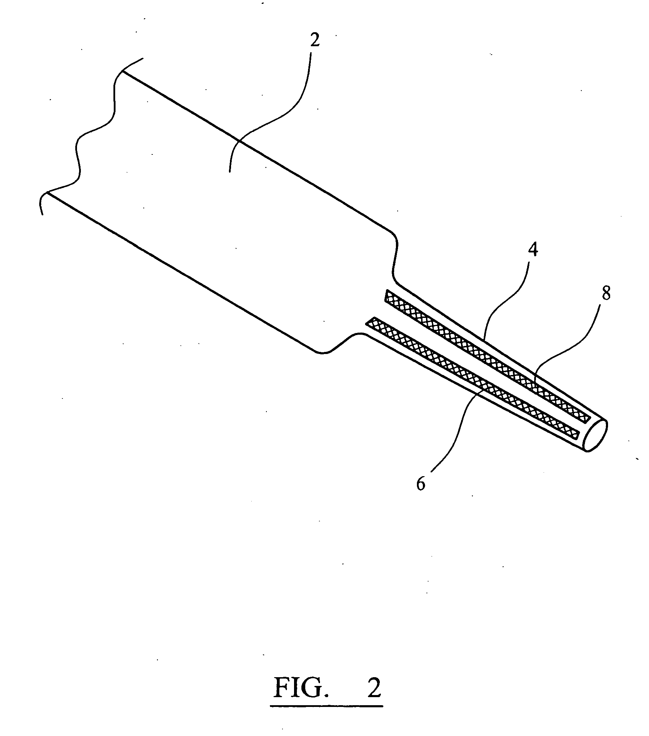

Figure 2 shows a syringe which includes a syringe body 2 and a delivery tube 4 through which cement within the body of the syringe can be delivered to a bone cavity. The syringe body can have an externally threaded spigot on its delivery end and the tube can have an internally threaded cap which can fit on to the spigot. Cement can be displaced from within the syringe body by means of a piston (not shown) which can slide along the body towards the end at which the delivery tube is fitted.

The syringe can be used as a mixing container for the cement, for example as discussed in the patent documents referred to above.

The delivery tube has formed in it a plurality of stripes 6, 8. Each of the stripes contains a thermochromic indicator material. The material can provide a visible indication of the temperature of the cement in the tube reaching a level which is characteristic of a critical stage in the cure of the cement, by changing colour. Each of the stripes is provided by a blend of a polymer and the indicator material. The tube is formed by coextrusion of the polymer/indicator material, and the polymer of the tube without indicator material. The stripes 6, 8 can contain different thermochromic indicator materials so that the tube can provide indications of different points in the curing process for a cement.

It is an advantage of the invention that a residue of cement will inevitably remain in the delivery tube after supply of cement to a bone cavity because a piston within the syringe body can only displace cement from within the body until the piston reaches the end of its stroke. The cement within the tube is therefore able to indicate the extent of cure throughout the subsequent period in which the cement is curing within the bone cavity.

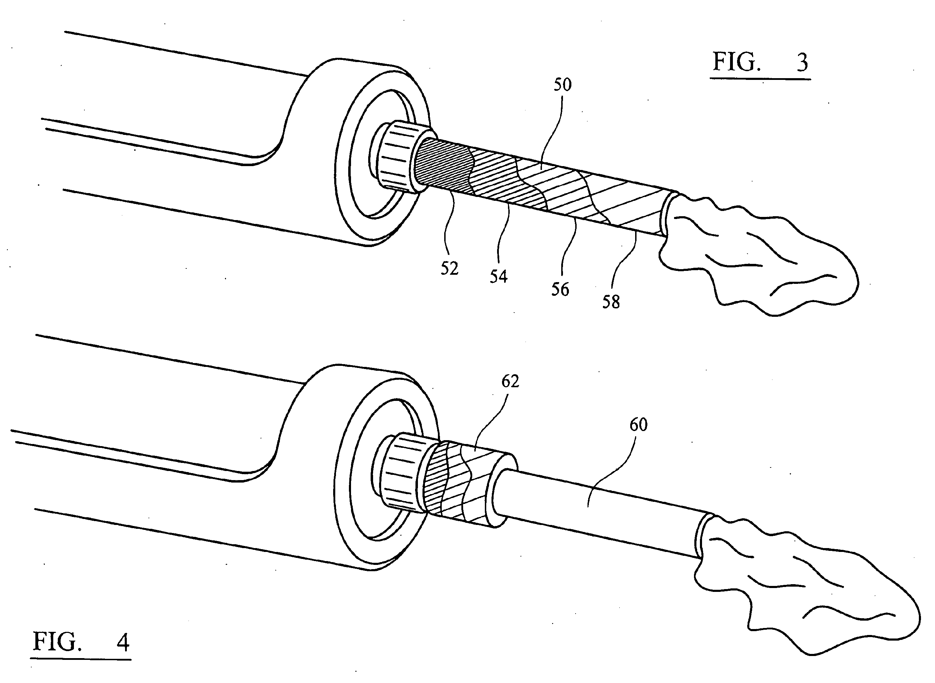

Figure 3 shows the nozzle 50 of a syringe device which can be used to deliver a bone cement, in particular into the medullary cavity of a patient's bone. Such syringe devices are well known. It is known for example that they can be used to provide a chamber in which the components of a bone cement can be mixed.

The nozzle 50 of the device shown in Figure 3 can be made from a material which includes in its composition a quantity of at least one thermochromic material, which exhibits a colour change when the temperature of a bone cement material within the nozzle reaches a predetermined level. It can be preferred for the material which is used to make the nozzle to include more than one thermochromic material so that colour changes are exhibited when the bone cement material reaches different predetermined levels. For example, colour changes might take place when the bone cement material reaches temperatures which correspond to the dough time, end of working time and setting time stages in the process of curing the cement. The syringe can be provided with a label which provides an indication of each of the colours of the material which is used to make the nozzle at each of those stages in the cement cure process. Figure 3 shows a nozzle in which different thermochromic materials are used in the material of the nozzle in different regions 52, 54, 56, 58 which are spaced apart along the length of the nozzle.

Figure 4 shows the nozzle 60 of a syringe device which can be used to deliver a bone cement, in particular into the medullary cavity of a patient's bone. The nozzle has a ring 62 on it. The ring is formed from a polypropylene which has been melt processed with a thermochromic material. The ring is a snug fit on the nozzle, for example as a result of being shrunk fit on to the nozzle, generally as a result of method which includes a step of heating the ring. Suitable techniques for shrink fitting an annular polymeric ring on to a cylindrical substrate are known.

## Claims

1. A device for use in the provision of bone cement to a patient, comprising a syringe body (2) **characterised in that** further comprises a tube (4) through which cement can be supplied from within the syringe body to a bone cavity, in which the tube comprises a polymer which is loaded with a thermochromic material.

2. A device as claimed in claim 1, in which the syringe includes a piston by which cement can be displaced from the syringe body to the bone cavity through the tube.

3. A device as claimed in claim 1, in which the polymer which is loaded with the thermochromic material is used to form the tube by a moulding process.

4. A device as claimed in claim 1, in which the amount of the thermochromic material in the polymer differs between different regions of the tube.

5. A device as claimed in claim 4, in which the thermochromic material is provided in one or more stripes (6,8) extending along at least part of the length of the tube.

6. A device as claimed in claim 5, in which the amount of thermochromic material in the material of the tube between first and second ones of the stripes is less than the amount of thermochromic material in the said first and second stripes.

7. A device as claimed in claim 1, in which the said polymer comprises a polyolefin.

8. A kit which comprises a device as claimed in claim 1 and a quantity of a bone cement material.

## Patentansprüche

1. Vorrichtung zur Verwendung bei der Verabreichung von Knochenzement an einen Patienten, mit einem Spritzenkörper (2), **dadurch gekennzeichnet, dass** sie weiter ein Rohr (4) aufweist, durch das Zement aus dem Spritzenkörper in eine Knochenhöhlung geliefert werden kann, wobei das Rohr ein Polymer aufweist, das mit einem thermochromen Material beschickt ist.

2. Vorrichtung nach Anspruch 1, bei der die Spritze einen Kolben umfasst, mit dem Zement aus dem Spritzenkörper in die Knochenhöhlung durch das Rohr verlagert werden kann.

3. Vorrichtung nach Anspruch 1, bei der das Polymer, das mit dem thermochromen Material beschickt ist, verwendet wird, um das Rohr in einem Formgussprozess zu bilden.

4. Vorrichtung nach Anspruch 1, bei der die Menge des thermochromen Materials in dem Polymer in unterschiedlichen Bereichen des Rohrs verschieden ist.

5. Vorrichtung nach Anspruch 4, bei der das thermochrome Material in einem oder mehreren Streifen (6, 8) zur Verfügung gestellt ist, die sich entlang wenigstens einem Teil der Länge des Rohres erstrecken.

6. Vorrichtung nach Anspruch 5, bei der die Menge an thermochromem Material in dem Material des Rohres zwischen ersten und zweiten der Streifen kleiner ist als die Menge an thermochromem Material in den ersten und den zweiten Streifen.

7. Vorrichtung nach Anspruch 1, bei der das Polymer ein Polyolefin aufweist.

8. Basisausrüstung, die eine Vorrichtung nach Anspruch 1 und eine Menge eines Knochenzementmaterials aufweist.

## Revendications

1. Dispositif destiné à délivrer un ciment osseux chez un patient, comprenant un corps de seringue (2), **caractérisé en ce qu'**il comprend en outre un tube (4) par l'intermédiaire duquel le ciment peut être délivré à partir de l'intérieur du corps de seringue jusqu'à une cavité osseuse, dans lequel le tube comprend un polymère qui est chargé avec un matériau thermochromique.

2. Dispositif selon la revendication 1, dans lequel la seringue comprend un piston qui permet de déplacer le ciment à partir du corps de seringue jusqu'à la cavité osseuse par l'intermédiaire du tube.

3. Dispositif selon la revendication 1, dans lequel le polymère qui est chargé avec le matériau thermochromique est utilisé de façon à former le tube par un processus de moulage.

4. Dispositif selon la revendication 1, dans lequel la quantité de matériau thermochromique dans le polymère diffère entre différentes régions du tube.

5. Dispositif selon la revendication 4, dans lequel le matériau thermochromique est délivrée sous la forme d'une ou de plusieurs bandes (6, 8) qui s'étendent le long d'une partie au moins de la longueur du tube.

6. Dispositif selon la revendication 5, dans lequel la quantité de matériau thermochromique dans le matériau du tube entre des première et deuxième bandes est inférieure à la quantité de matériau thermochromique dans lesdites première et deuxième bandes.

7. Dispositif selon la revendication 1, dans lequel ledit polymère comprend une polyoléfine.

8. Kit qui comprend un dispositif selon la revendication 1 et une quantité de matériau de ciment osseux.
